# EUROPEAN PATENT SPECIFICATION

(11) **EP 2 890 646 B1**
(45) Date of publication and mention of the grant of the patent: **14.09.2016**
(21) Application number: 13792742.2
(22) Date of filing: 28.08.2013
(51) Int. Cl.: C02F 3/34, C12R 1/125, C12R 1/40, C12N 1/20

(54) **BIO-INOCULANT AND USE THEREOF FOR TREATMENT OF EFFLUENTS**
BIOIMPFMITTEL UND VERWENDUNG ZUR BEHANDLUNG VON ABWASSER
BIO-INOCULANT ET SON UTILISATION POUR LE TRAITEMENT D'EFFLUENTS

(30) Priority: 29.08.2012 IN MM25052012
(43) Date of publication of application: 08.07.2015
(73) Proprietor: Indian Oil Corporation Ltd, Mumbai 400 051 Maharashtra (IN)
(72) Inventor: KUMAR, Manoj, Haryana 121007 (IN); SINGH, Mahendra, Pratap, Haryana 121007 (IN); TULI, Deepak, Kumar, Haryana 121007 (IN); MALHOTRA, Ravinder, Kumar, Haryana 121007 (IN)
(74) Representative: Oficina Ponti, SLP
(86) International application number: PCT/IB2013/058056
(87) International publication number: WO 2014/033638

(56) References cited:
- WO-A2-2011/161695
- US-A1- 2002 179 525
- US-A1- 2007 205 149
- US-A1- 2009 325 271
- US-A1- 2010 274 069

## Description

### FIELD OF INVENTION

The present invention relates to a bio-inoculant and its use for reduction of contaminants in effluents from processing industries.

### BACKGROUND OF THE INVENTION

Petroleum processing industries produce large quantity of water during various processing operations contaminated with hydrocarbons (oil and grease), phenols, heavy metals and in varying quantities depending upon the processing configuration of the plants. The refinery streams in addition to hydrocarbon and phenol contains other contaminants like sulphides, bezo (a) pyrene, heavy metals like Cr, V, Ni, Hg and cyanides in considerable amount.

These pollutants, if left untreated can cause serious ill effects to the environment, fauna and flora. Apart from this, if the water is to be re-used, it should be treated for removal of pollutants. The waste water in a typical petroleum processing industry is treated in Effluent Treatment Plant (ETP) by various physico-chemical methods in which the economically recoverable products like hydrocarbons are recovered and the rest pollutants are degraded by mainly microbial action.

Microbial degradation appears to be the most environment friendly approach of contaminant removal, as these microbes transform the pollutants to CO₂ and water. Typically, the biological treatment of the waste water is carried out in the biological treatment section by the activated sludge process. The success of the activated-sludge process is dependent upon establishing a mixed community of microorganisms that will remove and consume organic waste material and will be able to grow in under varying conditions. Due to adverse conditions, the unit does not run at its maximum efficiency and the treated wastewater still contains high oil and COD levels above the limits of recommended guidelines.

The efficiency of microbes in activated sludge process depends upon the concentration of pollutants, presence of heavy metals, temperature, pH and process conditions like mixing regime, loading rate, and the hydraulic flow rate. Effluent treatment plants are usually constrained to completely degrade the contaminants in the biological unit mainly due to lack of appropriate catabolic gene pool of microbial community. Hence, the efficiency of ETP can be improved by providing microbes with higher catabolic activity and degrade multiple contaminants in varying temperature and pH conditions and in presence of toxic heavy metals.

Moreover, the biological treatment of industrial wastewaters by activated sludge process is, however, often disrupted by shock load from organic (e.g., phenolic compounds, surfactants) and inorganic (e.g., heavy metals, sulfides etc.) chemicals present in the wastewater stream. Hence, it is desirable to have microbes which can tolerate these shocks.

US2009325271discloses a bio-assisted method for treatment of hydrocarbon contaminated soil employing novel microbes which are capable of decontaminating hydrocarbon contaminated soil having free flowing water or in slurry form or having large amount of gravels. The method comprises adding hydrocarbon releasing microbes followed by adding the microbes capable of degrading the released hydrocarbon to decontaminate the soil, wherein said microbes are grown separately in suitable vessels containing a suitable nutrient medium.

US7052901 discloses that fluids viscosified with viscoelastic surfactants (VESs) may have their viscosities reduced (gels broken) by the direct or indirect action of a biochemical agent, such as bacteria, fungi, and/or enzymes. The biochemical agent may directly attack the VES itself, or some other component in the fluid that produces a by-product that then causes viscosity reduction. The biochemical agent may disaggregate or otherwise attack the micellar structure of the VES-gelled fluid. The biochemical agent may produce an enzyme that reduces viscosity by one of these mechanisms. A single biochemical agent may operate simultaneously by two different mechanisms, such as by degrading the VES directly, as well as another component, such as a glycol, the latter mechanism in turn producing a by-product (e.g. an alcohol) that causes viscosity reduction. Alternatively, two or more different biochemical agents may be used simultaneously. In a specific, non-limiting instance, a brine fluid gelled with an amine oxide surfactant can have its viscosity broken with bacteria such as Enterobacter cloacae, Pseudomonas fluorescens, Pseudomonas aeruginosa, and the like.

Mandal et al. (2008) (In proceedings of 1st Intl. Conf. "Hazardous Waste Management", Chania, Crete, Greece: Vol. B2.4) teaches land forming for degradation of oily sludge using bacteria.

Sarma, A and Sarma, H (2010) Intl. J. Bot. Vol.6 (4), pages 441-448 discloses about addition of heavy metal like Cu and Mn which are required by bacteria for their growth improved the biodegradation.

Pala, A.I and Sponza, D.T (2010) Envir. Tech. Vol.17 (7), pages 673-685 discloses a single bacterium is used and the removal efficiencies of COD, TN and phenol for lower sludge ages were found higher for the *Pseudomonas sp.* added activated sludge system than that of the activated sludge system. Treatment efficiencies were found to be almost the same for both systems at high sludges.

Bujang et al. (2013), ARPN J. Agric. Biol. Sci. Vol. 8(2), Pages 108-115, discloses a single bacterium is use for degrading waste waters.

US6406882 provides an effective encapsulation system in form of coconut fibers for immobilizing consortia of microbes which can only degrade phenol.

Das, Nilanjana and Chandran, Preethy (2010), Biotech. Res. Intl. Vol. 2011, Article ID 941810, pages 1-13 is a review which discusses upon various issues related to hydrocarbon degradation, in general.

Wang et al (2010), J. Envir. Sci. Vol. 22(3), pages 381-388 relates to a specific stainless steel as biofilm carriers based bioreactor is required for higher activity of consortia.

WO2012137220 relates to bio-augmentation composition for improving hydrocarbons degradation efficiency of effluent treatment plant for hydrocarbon degradation in waste water generated from hydrocarbon processing industry and a method thereof.

Therefore there is a need in the art to have a bio-inoculant having multiple contaminant degradation ability in presence of toxic heavy metals at varying temperature and pH condition.

In light of the above, there is a need for an improved gene pool i.e. microbes, for further degradation to attain sustained expression

### SUMMARY OF THE INVENTION

Accordingly the main embodiment of the present invention provides a bio-inoculant composition comprising consortia of microorganisms selected from group of *Bacillus sp.* (IOC-EP-1), *Pseudomonas putida* (IOC-EP2), *Bacillus subtilis* (IOC-EP3) and *Pseudomonas putida* (IOC-EP4).

Another embodiment of the present invention provides a method of isolation of microbes from refinery waste water, said method comprising the steps of:
(a) inoculating liquid nutrient medium with the waste water from aeration tank of ETP in 5:1 to 5: 0.01 ratio and incubating at 35°C for 2 days;
(b) re-inoculating 1-5% of culture medium of step (a) in fresh medium containing crude oils and incubating for 2 days
(c) repeating the step (b) for eight cycles
(d) plating diluted culture medium of step (c) on BSM agar plates containing crude oils; and
(e) selecting the microbes having higher and faster contaminant degradation capability.

Another embodiment of the present invention provides a method of preparing a bio-inoculant composition comprising consortia of microbes, said method comprising the steps of:
(a) growing the individual colonies of microbes on agar medium;
(b) transferring a single loop of microbes grown in step (a) to a bioreactor containing basic nutrient medium;
(c) mixing the individual microbes grown in step (b) in equal ratio of 1: 1: 1: 1; and
(d) preparing a bio-inoculant composition by adsorbing the mixed microbes of step (c) on rice husk in the ratio of 1:9 (v/w).

Yet another embodiment of the present invention provides a method for improving efficiency of treatment of waste water from Effluent treatment plants (ETP), said method comprising the steps of:
(a) adding bio-inoculant composition comprising consortia of microorganisms in the waste water;
(b) adding optimized nutrients to wastewater
(c) subjecting the waste water to be treated by the consortia of microorganisms of step (a); and
(d) obtaining treated waste water having reduced contaminants.

### DETAILED DESCRIPTION OF THE INVENTION

While the invention is susceptible to various modifications and/or alternative processes and/or compositions, specific embodiment thereof has been shown by way of example in the drawings and tables and will be described in detail below. It should be understood, however that it is not intended to limit the invention to the particular processes and/or compositions disclosed, but on the contrary, the invention is to cover all modifications, equivalents, and alternative falling within the spirit and the scope of the invention as defined by the appended claims.

The tables and protocols have been represented where appropriate by conventional representations, showing only those specific details that are pertinent to understanding the embodiments of the present invention so as not to obscure the disclosure with details that will be readily apparent to those of ordinary skill in the art having benefit of the description herein.

The following description is of exemplary embodiments only and is not intended to limit the scope, applicability or configuration of the invention in any way. Rather, the following description provides a convenient illustration for implementing exemplary embodiments of the invention. Various changes to the described embodiments may be made in the function and arrangement of the elements described without departing from the scope of the invention.

The terms "comprises", "comprising", or any other variations thereof, are intended to cover a non-exclusive inclusion, such that one or more processes or composition/s or systems or methods proceeded by "comprises... a" does not, without more constraints, preclude the existence of other processes, sub-processes, composition, sub-compositions, minor or major compositions or other elements or other structures or additional processes or compositions or additional elements or additional features or additional characteristics or additional attributes.

### Definitions:

For the purposes of this invention, the following terms will have the meaning as specified therein:
As used herein, the term "*Bio*-*inoculant*", when used in the context of the present invention refers to consortium of selected microbes, when added to the ETP result in higher degradation of contaminant particularly hydrocarbons, phenols, sulfides and benzo (alpha) pyrene in presence of toxic heavy metals.

As used herein, the term *"Consortia or Consartium*" when used in the context of the present invention refers to combination of selected microbes resulting in higher degradation of contaminants in comparison to individual microbes

As used herein, the term "*Mixed liquor suspended solids (MLSS)"* when used in the context of the present invention refers to the concentration of suspended solids, in an aeration tank during the activated sludge process, which occurs during the treatment of waste water. MLSS is an important part of the activated sludge process to ensure that there is a sufficient quantity of active biomass available to consume the applied quantity of organic pollutant at any time. This is known as the food to mass ratio, more commonly notated as the F/M ratio. By maintaining this ratio at the appropriate level the biomass will consume high percentages of the food. This minimizes the loss of residual food in the treated effluent.

The present invention provides for novel composition of bacterial bio-inoculants. The bacteria were isolated from refinery waste water using selective enrichment techniques from Aeration tank of Indian Oil Guwahati Refinery, Guwahati, India. The composition of the present invention is bio-inoculant culture consortia of four bacteria, namely *Bacillus sp.* (referred to herein as IOC-EP-1) which was deposited with Microbial Type Culture Collection (MTCC), IMTECH, Chandigarh, India under Budapest Treaty on October 23, 2013 and given accession number MTCC-5870; *Pseudomonas putida* (referred to herein as IOC-EP2) which was deposited with MTCC, IMTECH, Chandigarh, India under Budapest Treaty on October 11, , 2013 and given accession number MTCC-5869; ; *Bacillus subtilis* (referred to herein as IOC-EP-3) which was deposited with MTCC, IMTECH, Chandigarh, India under Budapest Treaty on September 2, 2013 and given accession number MTCC-5851; and *Pseudomonas putida* (referred to herein as IOC-EP4) which was deposited with MTCC, IMTECH, Chandigarh, India under Budapest Treaty on October 23, 2013 and given accession number MTCC-5871.

The bio-inoculant composition is a consortium of selective microorganisms enabling effective degradation of contaminants present in wastewater.

The bacterial microbes were isolated from refinery waste water using selective enrichment techniques. Those microbes which give higher and fast biodegradation ability were selected and formulated into a bio-inoculant composition. The bio-inoculant of present invention is developed based on the growth ability of the isolated bacteria and to take advantage of combined effect of microbial metabolism. The bio-inoculant consortia of present invention are unique as they have unexpectedly shown to improve efficiency in treatment of waste water for reduction of hydrocarbons, phenols, sulfides and benzo (alpha) pyrene.

The bacteria bio-inoculant consortia of present invention can work in wide range of pH (5-9), temperature (10-50°C) and hydrocarbon loading (0.0005%-5% or 5 ppm-5%). The bio-inoculant of the present invention improves the efficiency of activated sludge in effluent treatment plant for reduction of oil and grease content (hydrocarbon), phenol, sulphides and benzo (a) pyrene content and other contaminants. The bio-inoculant has the ability to degrade phenol (contamination concentration 2000 ppm), sulphides (contamination concentration 5000 ppm) and bezo (a) pyrene (contamination concentration 100 ppm), hydrocarbons(oil and grease) (contamination concentration 5%) in presence of heavy metals like Cr, V, Ni, Hg and cyanides.

The microbes of present invention have high ability of self-propagating at concentration of 10²cfu/ml in wastewater. Hence, the mixture of microbes may not require any encapsulation or immobilization on any matrix and continuous dosing of bio-inoculant. The bio-inoculant can be dosed in aeration tank or other biological treatment unit in a way similar to chemical dosing used commonly in refinery ETP without any special provision or device.

The bio-inoculant consortium also has the capability of producing biosurfactant which enables efficient solubilization and removal of contaminants. More specifically the consortium is unique mixture of wherein one of the selected microbes has capability of producing biosurfactant (*Pseudomonas putida* referred to herein as IOC-EP2).

The bio-inoculant consortium is immobilized on rice husk previously UV sterilized in ratio 1:9 (volume per weight). This material was packed in UV sterilized plastic bag keeping 30% space for air and it was sealed.

The present invention also provides for a process reducing contaminants in the waste waters released from industries effluent treatment plants (ETP) by using the novel bio-inoculant composition.

The present invention also relates to a process of improving efficiency of treatment of waste water for reduction of hydrocarbons, phenols, sulfides and benzo (alpha) pyrene therein said method comprising steps of:
a) preparing a bio-inoculant consortium composition capable of degrading hydrocarbons in waste water wherein microorganisms in the bio-inoculant are adsorbed on a biodegradable carrier, and
b) subjecting the bio-inoculant consortium to the hydrocarbon contaminated waste water followed by addition of optimized nutrients to promote hydrocarbon degradation.

Further, at least one of microbes present in the bio-inoculant has the ability to produce biosurfactant to accelerate the biodegradation activity.

Accordingly the main embodiment of the present invention provides a bio-inoculant composition comprising consortia of microorganisms selected from group of *Bacillus sp.* (IOC-EP-1), *Pseudomonas putida* (IOC-EP2), *Bacillus subtilis* (IOC-EP-3) and *Pseudomonas putida* (IOC-EP4).

Another embodiment of the present invention provides a bio-inoculant composition, wherein bio-inoculant composition has ability to efficiently treat waste water of effluent treatment plants (ETP).

Another embodiment of the present invention provides a bio-inoculant composition, wherein the bio-inoculant composition has ability to degrade and efficiently reduce the hydrocarbons, phenols, sulphides and benzo(alpha) pyrene present in the ETP.

Another embodiment of the present invention provides a bio-inoculant composition, wherein the bio-inoculant composition has ability to degrade and efficiently reduce hydrocarbons, phenols, sulphides and benzo(alpha) pyrene present in the ETP in the range of 95-99%.

Yet another embodiment of the present invention provides a bio-inoculant composition, wherein the composition comprises equal ratio of individual microbes

Another embodiment of the present invention provides a bio-inoculant composition, wherein the microbes have high ability of self-propagating at concentration of 10²cfu/ml in ETP waste water.

Another embodiment of the present invention provides a method of isolation microbes from refinery waste water, said method comprising the steps of:
(a) inoculating liquid nutrient medium with waste water from aeration tank of ETP in 5:1 to 5:0.01 ratio and incubating at 35°C for 2 days;
(b) re-inoculating 1-5% of culture medium of step (a) in fresh medium containing crude oils and incubating for 2 days;
(c) repeating the step (b) for eight cycles;
(d) plating diluted culture medium of step (c) on BSM agar plates containing crude oil; and
(e) selecting the microbes having higher and faster contaminants degradation capability.

Another embodiment of the present invention provides a method wherein the liquid medium of step (a) comprises of mineral salt media, BSM along with salts of Cr, V, Ni, Hg, and cyanides in the range of 1-100 ppm.

Yet another embodiment of the present invention provides a method wherein the liquid medium of step (b) contains about 5% w/v of crude oil.

Another embodiment of the present invention provides a method, wherein the microorganisms are selected from group comprising of *Bacillus sp.* IOC-EP-1, *Pseudomonas putida* IOC-EP2, *Bacillus subtilis* IOC-EP3 and *Pseudomonas putida* IOC-EP4.

Another embodiment of the present invention provides a method of preparing a bio-inoculant composition comprising consortia of microbes, said method comprising the steps of:
(a) growing the individual colonies of microbes on agar medium;
(b) transferring a single loop of microbes grown in step (a) to a bioreactor containing basic nutrient medium;
(c) mixing the individual microbes grown in step (b) in equal ratio of 1: 1: 1: 1; and
(d) preparing a bio-inoculant composition by adsorbing the mixed microbes of step (c) on rice husk in the ratio of 1:9 (v/w).

Another embodiment of the present invention provides a method, wherein the bio-inoculant composition comprise of microorganisms selected from group of *Bacillus sp.* IOC-EP-1, *Pseudomonas putida* IOC-EP2, *Bacillus subtilis* IOC-EP3 and *Pseudomonas putida* IOC-EP4.

Yet another embodiment of the present invention provides a method for improving efficiency of treatment of waste water from Effluent treatment plants (ETP), said method comprising the steps of:
(a) adding bio-inoculant composition comprising consortia of microorganisms in the waste water;
(b) adding optimized nutrients to wastewater
(c) subjecting the waste water to be degraded by the consortia of microorganisms of step (a); and
(d) obtaining treated waste water having reduced contaminants.

Another embodiment of the present invention provides a method wherein the bio-inoculant composition comprises of microorganism consortia selected from group comprising of *Bacillus sp.* IOC-EP-1, *Pseudomonas putida* IOC-EP2, *Bacillus subtilis* IOC-EP3 and *Pseudomonas putida* IOC-EP4.

Another embodiment of the present invention provides a method wherein bio-inoculant microorganism consortia efficiently reduces the hydrocarbons, phenols, sulphides and benzo(alpha) pyrene present in the waste water of ETP.

Yet another embodiment of the present invention provides a method, wherein bio-inoculant microorganism consortia reduces hydrocarbons, phenols, sulphides and benzo(alpha) pyrene present in the ETP in the range of 95-99%.

Yet another embodiment of the present invention provides a method wherein the microbes have high ability of self-propagating at concentration of 10²cfu/ml in ETP waste water.

Yet another embodiment of the present invention proA method as claimed in any one of claims 13-16, wherein the optimized nutrients are selected from the group comprising of yeast extract 1-0.01 g/l; KNO₃ 0.5-0.2 g/l; di-ammonium phosphate 0.5-0.2 g/l and biotin and folic acid 0.1-0.001 g/l.

In accordance with another embodiment of the present invention, the bio-inoculant reduce in the range of 95%-98% phenol, sulphides, bezo (a) pyrene, oil and grease, respectively in presence of heavy metals. Similar trends of degradation was achieved when degradation of phenol, sulphides, bezo (a) pyrene, oil and grease was evaluated in absence of salts containing Cr, V, Ni, Hg and cyanides. In the subsequent experiments, the mixture of phenol, sulphides, bezo (a) pyrene, oil and grease was evaluated under similar experiment conditions. Subsequently, it was evaluated under Modified Strum test conditions.

After efficacy tests at laboratory scale the microbial consortia/blend are evaluated in a bioreactor (simulating the conditions of aeration tank) by using wastewaters obtained from outlet of aeration tank of refineries having contaminates sulphides, bezo (a) pyrene, oil and grease and Cr, V, Ni, Hg and cyanides in various concentrations (Table 2).

According to the present invention, bio-inoculant were evaluated for their potential to reduce oil and grease content of wastewater from inlet of aeration tank of ETP of a typical hydrocarbon processing industry in bioreactor under various pH (6-9) and temperature (10-50 degree C) conditions. The results showed that bio-inoculant could reduce in the range of 96-99% of phenol, sulphides, bezo (a) pyrene, oil and grease, respectively in 12hrs from wastewater from refinery effluent treatment plant under various temperature (10-50°C) and pH regime (6-9). This bio-inoculant could improve mixed liquor suspended solids (MLSS) as well the efficiency of aeration tank in terms of phenol, sulphides, bezo (a) pyrene, oil and grease content removal significantly. It was found that once the microbial population of microbial blend in a system is stabilized, no dosing of the microbial blend is required. It is also tolerant for hydrocarbons shock loading, pH and temperature.

This bio-inoculant/microbial blend can be effectively used for improving the efficiency of ETP in terms the reduction of oil and grease, phenol and other contaminants in hydrocarbon refinery effluent, petrochemical plants and in any other industry having effluent with these contaminants.

The following non-limiting examples illustrate specific embodiments of the present invention. They are not intended to be limiting the scope of the present invention in any way.

The invention will now be explained with the help of following examples. However, the scope of the invention should not be limited to these examples as the person skilled in the art can easily vary the proportion of the ingredients and combinations.

### EXAMPLES

### EXAMPLE 1:

### Isolation of bacteria capable of degrading various contaminants, development of microbial blend and evaluation to decontaminate wastewater

Microbes were also isolated by using standard enrichment technique from activated sludge from aeration tank of ETP of the refinery. The nutrient media (Mineral salt media, BSM) used for enrichment process includes (grams per liter) (g per liter) KH₂PO₄ (1.0), K₂HPO₄ (0.8), MgSO₄ (1.0), (NH4)₂SO₄ (0.50), KNO₃ (0.55), Trace element (5 ml) and Multi vitamin solution (0.5) and 5% crude oil as sole energy and carbon source. Trace element solution contained (milligrams per liter), 23 mg of MnCl₂· 2H₂O, 30 mg of MnCl₄·H₂O, 31 mg of H₃BO₃, 36 mg of CoCl₂·6H₂O, 10 mg of CuCl₂·2H₂O, 20 mg of NiCl₂·6H₂O, 30 mg of Na₂MoO₄·2H₂O and 50 mg ZnCl₂ (pH 7.0). The multivitamin solution (g/l) includes Biotin (0.01), Folic acid (0.01), Pyridoxine HCl (0.2), Thiamine HCl (0.06), Riboflavin (0.04), Nicotinic acid (0.01), Ca-Pentotheonate (0.01), Lipoic acid (0.0075), Vitamin B12 (0.0005), PABA. The nutrient system also contained salts of Cr, V, Ni, Hg and cyanides (individually 1 ppm-100 ppm and in different combinations, up to 1%) to select the microbes having tolerance to these contaminants. In this media, the waste water from aeration tank of ETP was taken as inoculum (in the ratio of 5:1 to 5:0.01) and incubated at 35°C on a rotary shaker (200 rpm) for two days. After two days 1-5% of the culture medium was transferred to fresh media containing crude oil (5%, weight per volume) and re-incubated for another two days. The crude oil used for the purpose as herein is unique since it has a low API density, high concentration of metalloprohyrins and high aromaticity. Following eight cycles of such enrichment, 1 ml of the culture was diluted and plated on BSM agar plates containing the crude oil. The bacterial colonies obtained were further purified, by streaking on nutrient agar plate (peptone 5.0 g/l, yeast extract 5 g/lm NaCl 2.5% and 2% agar). The bacteria having higher and fast contaminats degradation ability under above experimental conditions were selected, purified and characterized for their taxonomic identifications.

The bio-inoculant is made up of the following organisms: *Bacillus Sp*.IOC-EP-1, *Pseudomonas putida* IOC-EP2, *Bacillus subtilis* IOC-EP-3, *Pseudomonas putida* IOC-EP4, deposited with Microbial Type Culture Collection and Gene Bank (MTCC), Sector 39-A, Chandigarh-160036, India.

### EXAMPLE 2: Degradation by Bio-inoculant

The bio-inoculant of the present invention was developed based on the growth ability of the isolated bacteria and to take advantage of synergy of microbial metabolism. The four microbes are combined to make the bio-inoculant used in present invention. The degradation potential of the microbial consortium is examined in Shake flask and Modified Strum test (CO₂ evolution measurement). In the shake flask the bio-inoculant could reduce around 95%, 96%,97%, 98% phenol, sulphides, bezo (a) pyrene, oil and grease, respectively in shake flask in presence of heavy metals while the result of Modified Strum test have shown biodegradability of contaminates more than 90% using the microbial consortium as inoculum.

For evaluation and use, the bio-inoculum is prepared by first growing the individual microbe on separate agar plates in a conventional manner. Subsequently, growing them individually in a bioreactor containing a nutrient solution comprising inorganic and organic nitrogen, phosphorus source, and trace metals in different concentrations in a bioreactor. The bioreactor preferably contains control devices for temperature, pH, agitation, aeration and stirring. Particularly preferred nutrient system for growth includes (g per litre) KH₂PO₄ (0.8), K₂HPO₄ (1.0), MgSO₄ (0.1), (NH4)₂SO₄ (0.05), KNO₃ (0.19), Trace element (3 ml solution) and Multi vitamin solution (2 ml), molasses 5%. The trace element solution (gram per litre) comprises Nitrilotriacetic acid (0.1), FeSO₄.7H₂O (0.15), MnCl₂.4H₂O (0.002), CoCl₂.6H₂O (0.02), CaCl₂.2H₂0 (0.05), ZnCl₂ (0.07), CuCl₂.H₂O (0.02), H₃BO₃ (0.002), Na₂MoO₄ (0.001), Na₂SeO₃ (0.02), NiSO₄ (0.01-0.03), SnCl₂ (0.01). The multivitamin solution (g/l) includes Biotin 0.03, Folic acid (0.03), Pyridoxine HCl (0.5), Thiamine HCl (0.02), Riboflavin (0.02), Nicotinic acid (0.005), Ca-Pentotheonate (0.009), Lipoic acid (0.004), Vitamin B12 (0.001), PABA (0.0029). After appropriate growth of individual bacteria, they were mixed in equal ratio to prepare the bio-inoculant.

The appropriately grown mixed microbial culture of the invention was adsorbed on rice husk previously UV sterilized in ratio 1:9 (volume per weight). This material was packed in UV sterilized plastic bag keeping 30% space for air and it was sealed.

The laboratory developed microbial consortia along with optimized nutrients was evaluated for its potential to reduce O&G content of wastewater in shake flask. Along with the bio-inoculant, wastewater was supplemented with nutrients i.e., yeast extract 0.01g/l, urea : 0.01g/l, KNO₃ : 0.2 g/l, Ammonium phosphate : 0.2 g/l, biotin and folic acid : 0.001g/l. The basic experimental design involves following flask in triplicate:
Control: wastewater to be tested
Non-augmented test: wastewater with activated sludge
Nutrient amended test: wastewater with activated sludge and nutrients
Augmented test: wastewater with activated sludge and nutrients and 10² cfu/ml of bio-inoculant

The wastewater was also amended to with salts of Cr, V, Ni, Hg and cyanides (individually 100 ppm) to see the effect of these toxic materials along with control without these contaminants. The flasks were incubated at 35°C in shaking condition for 12 hr. It was observed that bio-inoculant was able to degrade contaminants in presence as well as in absence equally. Table-1 shows the hydrocarbon degradation.

**Table 1- Degradation by bio-inoculant**

| **S.No.** | **Test system** | **% degradation in 12 hr** | | | |
|---|---|---|---|---|---|
| | | **O&G content** | **Phenol** | **Sulphide** | **Benzo(alpha) pyrene** |
| 1 | Control | 10 | 17 | 12 | 07 |
| 2 | Non-augmented test | 42 | 40 | 28 | 29 |
| 3 | Nutrient amended test | 50 | 60 | 39 | 43 |
| 4 | Augmented test along with nutrient | 98 | 99 | 95 | 98 |

### EXAMPLE 3: Evaluation of the microbial preparation in bioreactor

The bio-inoculant was evaluated in a bioreactor of 7 liter capacity containing 5 liter wastewater along with activated sludge. pH of reactor was in range of 5-9. The bioreactor was amended with nutrient mixture, urea, di-bio-inoculant. Reactor was maintained in the continuous mode and 400 rpm. The bioreactor continuously feeded with untreated wastewater in the manner that after every 12 hrs the total content of the bioreactor replaced.

The time dependency reduction of the contaminants were tested wherein at zero hour sample was withdrawn for measuring the O&G, phenol, sulphide, benzo(alpha)pyrene content, and similar samples were withdrawn at every 12 hrs. All samples were drawn in triplicate. This time dependency reduction in contaminants was tested in the bioreactor over the period of 90 days. The results are shown in accompanying Table -2. Table-2 also gives data of non-augmented test and result indicates effectiveness of the addition of bio-inoculant and optimized nutrients.

Once stabilized, introduced bacteria could survive for at least two years. Improvement in MLSS content is shown in Table-3 in comparison to non-augmented test. This augmented growth with nutrient medium provides that the bacterial consortia with better growth and also enables efficient degradation of waste water effluents.

Further the bacteria consortia were given different shock conditions to match the fluctuating natural environmental conditions. The shock conditions comprised providing the bacteria with sudden higher hydrocarbons having a concentration of around 10%, pH 3-12 and temperature up to 65°C. Surprisingly it was found that the bacterial consortia could revive within 48 hrs to cell count of 10⁶ CFU/ml (Table-4) and has surprisingly faster recovery. Thus the bacterial consortium of present invention is unique such that it can withstand temperature and/or pH fluctuations which are commonly occur in natural environmental conditions.

**Table 2: Time dependent reduction in contaminant content in bioreactor**

| **O&G content (ppm)** | | **Phenol (ppm)** | | **Sulphide (ppm)** | | **Benzo(alpha)Pyrene (ppm)** | |
|---|---|---|---|---|---|---|---|
| **Control** | **Experiment** | **Control** | **Experiment** | **Control** | **Experiment** | **Control** | **Experiment** |
| 68 | 68 | 5.9 | 5.9 | 10.6 | 10.6 | 7 | 7 |
| 63 | 8.6 | 4.0 | 2 | 9.5 | 4.5 | 6.3 | 2.3 |
| 66.5 | 8.1 | 4.3 | 1.3 | 9.2 | 2 | 6.0 | 1 |
| 59.3 | 4.1 | 4.2 | 0.22 | 9.0 | 0.32 | 6.3 | 1.2 |
| 50.1 | 2.6 | 3.2 | 0.02 | 8.3 | 0.12 | 6.1 | 0.21 |
| 45.3 | 2.2 | 3.2 | 0.2 | 7.5 | 0.06 | 6.0 | 0.12 |
| 36.2 | 1.8 | 3.2 | 0.03 | 7.4 | 0.07 | 5.1 | 0.22 |
| 33.2 | 1.9 | 3.1 | 0.2 | 7.1 | 0.12 | 5.2 | 0.29 |
| 33.1 | 3.4 | 3.0 | 0.04 | 7.0 | 0.15 | 5.2 | 0.31 |
| 32.6 | 2.8 | 3.01 | 0.14 | 7.1 | 0.16 | 5.1 | 0.12 |
| 38.2 | 4.1 | 3.1 | 0.1 | 6.9 | 0.17 | 5.0 | 0.27 |
| 41.8 | 2.1 | 3.1 | 0.02 | 6.0 | 0.12 | 5.5 | 0.5 |

**Table-3 Mixed Liquid Suspended Solids (MLSS) improvement**

| **Day** | **MLSS (ppm)** | |
|---|---|---|
| | **Augmented test along with nutrient** | **Non-augmented test** |
| 0 | 1200 | 1200 |
| 5 | 1800 | 1700 |
| 10 | 2000 | 1750 |
| 15 | 2600 | 1700 |
| 20 | 3550 | 1500 |
| 25 | 4000 | 1650 |
| 30 | 4200 | 1600 |
| 35 | 4250 | 1500 |
| 40 | 4500 | 1500 |
| 45 | 4200 | 1100 |
| 50 | 4230 | 1500 |
| 60 | 4500 | 1550 |
| 70 | 4290 | 1500 |
| 80 | 4350 | 900 |
| 90 | 4500 | 1100 |

**Table-4: Effect of shock loads on microbial count of bio-inoculant as well in non-augmented test**

| | **Augmented test along with nutrient** | | **Non-augmented test** | |
|---|---|---|---|---|
| **Day** | **O&G content (ppm)** | **Microbial count (CFU/ml)** | **O&G content (ppm)** | **Microbial count (CFU/ml)** |
| 0 | 15.0 | 5.6 × 10⁷ | 15.0 | 5.6 × 10⁷ |
| 1 | 4.1 | 9.3 × 10⁸ | 13.5 | 5.0 × 10⁷ |
| 2 | 2.9 | 7.2 × 10¹⁰ | 14.2 | 2.6 × 10⁸ |
| 5 | 0.5 | 4.1 × 10¹¹ | 12.2 | 5.8 × 10⁸ |
| 10* | 0.5 | 9.9 × 10¹¹ | 10.5 | 5.6 × 10⁹ |
| 11 | 8.9 | 1.9 × 10² | 14.9 | 1.3 × 10² |
| 12 | 2.3 | 3.9 × 10⁶ | 14.5 | 2.6 × 10² |
| 14 | 1.0 | 5.9 × 10¹¹ | 14.1 | 5.5 × 10³ |
| 16 | 0.5 | 3.2 × 10¹¹ | 14.4 | 7.9 × 10³ |

| | | | | |
|---|---|---|---|---|
| *Given shock load of pH 10 and Temperature of reactor 50°C on the 10^{th} day | | | | |

## Claims

1. A bio-inoculant composition comprising a consortia of microorganisms of *Bacillus sp*. IOC-EP-1, *Pseudomonas putida* IOC- EP2, *Bacillus subtilis* IOC-EP-3 and *Pseudomonas putida* IOC-EP4 in equal proportions which improves efficiency of the activated sludge process in effluent treatment plant (ETP) of a hydrocarbon processing industry in terms of reduction of hydrocarbons, sulphides, phenols and benzo (alpha) pyrene content when dosed at concentration of 10²cfu/ml in the aeration tank inlet.

2. A bio-inoculant composition as claimed in claim 1, has ability to improve the MLSS content of ETP.

3. A bio-inoculant composition as claimed in any one of claims 1-2, wherein the bio-inoculant composition has ability to degrade and efficiently reduce hydrocarbons, phenols, sulphides and benzo(alpha) pyrene present in the ETP in the range of 95-99%.

4. A bio-inoculant composition as claimed in claim-1 , has ability for quick recovery of the biological activity in aeration tank in case of plant disturb due to hydrocarbon shock load, temperature shock, pH shock and salinity shock.

5. A bio-inoculant composition as claimed in claim 1, has tolerance for pH ranging from 5-9 and temperature from 10-50 degree C and hydrocarbon loading up to 5%.

6. A bio-inoculant composition as claimed in claim 1, has tolerance for Cr, V, Ni, Hg, and cyanides in the range of 1-100 ppm.

7. A method for improving efficiency of treatment of waste water from Effluent treatment plants (ETP) using the bio-inoculant composition as claimed in claim 1, said method comprising the steps of :
a. adding bio-inoculant composition comprising consortia of microorganisms in the waste water;
b. adding optimized nutrients to waste water;
c. subjecting the waste water to be degraded by the consortia of microorganisms of step (a); and
d. obtaining treated waste water having reduced contaminants of hydrocarbons, phenols, sulphide and benzo(alpha) pyrene.

8. A method as claimed in claim 7, wherein the microbes have high ability of self-propagating at concentration of 10²cfu/ml in ETP waste water.

9. A method as claimed in claim 7, wherein the optimized nutrients are selected from the group comprising of yeast extract 1-0.01 g/l; KNO₃ 0.5-0.2 g/l; di-ammonium phosphate 0.5-0.2 g/l and biotin and folic acid 0.1-0.001g/l.

## Patentansprüche

1. Eine Bio-Impfmittelzusammensetzung, die Gruppierungen von Mikroorganismen von *Bacillus sp.* IOC-EP-1, *Pseudomonas putida* IOC-EP2, *Bacillus subtilis* IOC-EP-3 und *Pseudomonas putida* IOC-EP4 in gleichen Anteilen aufweist, was die Effizienz des Belebtschlammprozesses bei einer Abwasserbehandlungsanlage (ETP - effluent treatment plant) einer Kohlenwasserstoff verarbeitenden Industrie bezüglich einer Reduktion des Gehalts an Kohlenwasserstoffen, Sulfiden, Phenolen und Benzo(alpha)pyren bei einer Dosierung in einer Konzentration von 10² cfu/ml in dem Belüftungsbeckeneinlass verbessert.

2. Eine Bio-Impfmittelzusammensetzung gemäß Anspruch 1 weist die Fähigkeit auf, den Gehalt an Schwebstoffen im Belebtschlamm (MLSS-Gehalt, MLSS = mixedliquor suspended solids) von ETP zu verbessern.

3. Eine Bio-Impfmittelzusammensetzung gemäß einem der Ansprüche 1 bis 2, wobei die Bio-Impfmittelzusammensetzung die Fähigkeit aufweist, Kohlenwasserstoffe, Phenole, Sulfide und Benzo(alpha)pyren, die in der ETP vorliegen, im Bereich von 95 - 99% abzubauen und effizient zu verringern.

4. Eine Bio-Impfmittelzusammensetzung gemäß Anspruch 1 weist die Fähigkeit einer raschen Regeneration der biologischen Aktivität im Belüftungsbecken im Fall einer Anlagenstörung aufgrund einer Kohlenwasserstoff-Stoßbelastung, eines Temperaturschocks, eines pH-Schocks und Salinitätsschocks auf.

5. Eine Bio-Impfmittelzusammensetzung gemäß Anspruch 1 weist eine Toleranz bezüglich eines pH-Werts zwischen 5 und 9 und einer Temperatur zwischen 10 und 50 Grad C und einer Kohlenwasserstofflast bis zu 5% auf.

6. Eine Bio-Impfmittelzusammensetzung gemäß Anspruch 1 weist eine Toleranz bezüglich Cr, V, Ni, Hg und Cyaniden im Bereich von 1 bis 100 ppm auf.

7. Ein Verfahren zum Verbessern der Effizienz der Behandlung von Abwasser aus Abwasserbehandlungsanlagen (ETP) unter Verwendung der Bio-Impfmittelzusammensetzung gemäß Anspruch 1, wobei das Verfahren folgende Schritte aufweist:
a. Hinzufügen der Bio-Impfmittelzusammensetzung, die Gruppierungen von Mikroorganismen aufweist, in dem Abwasser;
b. Hinzufügen optimierter Nährstoffe zu Abwasser;
c. Beaufschlagen des Abwassers, das durch die Gruppierungen von Mikroorganismen des Schritts (a) abgebaut werden soll; und
d. Erhalten von behandeltem Abwasser, das verringerte Verunreinigungen von Kohlenwasserstoffen, Phenolen, Sulfid und Benzo(alpha)pyren aufweist.

8. Ein Verfahren gemäß Anspruch 7, bei dem die Mikroben bei einer Konzentration von 10² cfu/ml in ETP-Abwasser eine hohe Fähigkeit der Selbstvermehrung aufweisen.

9. Ein Verfahren gemäß Anspruch 7, bei dem die optimierten Nährstoffe aus der Gruppe ausgewählt sind, die von Hefeextrakt 1 - 0,01 g/l; KNO₃ 0,5 - 0,2 g/l; Diammoniumphosphat 0,5 - 0,2 g/l und Biotin und Folsäure 0,1 - 0,001 g/l aufweist.

## Revendications

1. Une composition de bio-inoculants comprenant des consortiums de microorganismes de *Bacillus sp.* IOC-EP-1, *Pseudomonas putida* IOC-EP2, *Bacillus subtilis* IOC-EP-3 et *Pseudomonas putida* IOC-EP4, dans des proportions égales, ce qui améliore l'efficacité du processus de boues activées à l'usine de traitement des effluents (*effluent treatment plant* (ETP)) d'une industrie de traitements des hydrocarbures en termes de réduction en teneur d'hydrocarbures, sulfures, phénols et benzo (alpha) pyrène lorsque dosés à une concentration de 10²cfu/ml à l'entrée du réservoir d'aération.

2. Une composition de bio-inoculants telle que revendiquée selon la revendication 1, possédant la capacité d'améliorer la teneur en MSLM de l'ETP.

3. Une composition de bio-inoculants telle que revendiquée selon une quelconque des revendications 1-2, où la composition de bio-inoculants possédant la capacité de dégrader et réduire efficacement les hydrocarbures, sulfures, phénols et benzo (alpha) pyrène présents dans l'ETP dans des proportions de 95-99%.

4. Une composition de bio-inoculants telle que revendiquée dans la revendication 1, possédant la capacité de permettre un rétablissement rapide de l'activité biologique dans le réservoir d'aération en cas de trouble de l'usine dû à un choc de charge d'hydrocarbures, choc de température, choc de pH et choc de salinité.

5. Une composition de bio-inoculants telle que revendiquée dans la revendication 1, dotée d'une tolérance à un pH compris entre 5 et 9, une température comprise entre 10 et 50 degrés °C et une charge en hydrocarbures allant jusqu'à 5%.

6. Une composition de bio-inoculants telle que revendiquée dans la revendication 1, dotée d'une tolérance au Cr, V, Ni, Hg, et aux cyanures compris entre 1 et 100 ppm.

7. Un procédé pour améliorer l'efficacité du traitement des eaux usées des usines de traitement des effluents (ETP) utilisant la composition de bio-inoculants telle que revendiquée selon la revendication 1, ledit procédé comprenant les étapes :
a. d'ajout d'une composition de bio-inoculants comprenant des consortiums de microorganismes dans les eaux usées ;
b. l'ajout de substances nutritives aux eaux usées ;
c. de soumission des eaux usées devant être dégradées par les consortiums de microorganismes selon l'étape (a) ; et
d. l'obtention des eaux usées traitées contenant des contaminants réduits d' hydrocarbures, phénols, sulfures et benzo(alpha) pyrène.

8. Un procédé tel que revendiqué selon la revendication 7, où les microbes possèdent une haute propension à se propager à une concentration de 10²cfu/ml dans les eaux usées d'ETP.

9. Un procédé tel que revendiqué selon la revendication 7, où les substances nutritives optimisées sont sélectionnées dans le groupe comprenant un extrait de levure 1-0.01 g/l ; KNO₃ 0.5-0.2 g/l ; phosphate de diammonium 0.5-0.2 g/l et biotine et acide folique 0.1-0.001 g/l.
